# EUROPEAN PATENT APPLICATION

(11) **EP 4 635 551 A1**
(43) Date of publication of application: **22.10.2025**
(21) Application number: 23917281.0
(22) Date of filing: 15.12.2023
(51) Int. Cl.: A61M 36/12, A61N 5/10

(54) **MEDICAMENT SUPPLY APPARATUS AND NEUTRON CAPTURE THERAPY SYSTEM**

(30) Priority: 19.01.2023 CN 202310073561
(71) Applicant: Neuboron Therapy System Ltd., Xiamen, Fujian 361026 (CN)
(72) Inventor: LIU, Yuanhao, Nanjing, Jiangsu 211112 (CN); GONG, Qiuping, Nanjing, Jiangsu 211112 (CN); GENG, Wenxiu, Nanjing, Jiangsu 211112 (CN)
(74) Representative: Gunzelmann, Rainer
(86) International application number: PCT/CN2023/139004
(87) International publication number: WO 2024/152814

(57) **Abstract**

One aspect of the present invention relates to a medicament supply apparatus applied to a neutron capture therapy system, including a medicament passing member, configured to supply a medicament to a to-be-irradiated body; and a containing mechanism, configured to contain the medicament passing member, the containing mechanism including a first container arranged on a partition wall. Another aspect of the present invention relates to a neutron capture therapy system including the above medicament supply apparatus. By designing the containing mechanism, the medicament passing member outside a shielding door can bypass the shielding door and directly enter an irradiation room. In a process of moving the to-be-irradiated body from a medicament control room to the irradiation room, medicament supplying does not need to be stopped, thereby achieving a purpose of continuously supplying the medicament to the to-be-irradiated body. By the arrangement of the containing mechanism, exposure of the medicament passing member in the irradiation room to a neutron irradiation environment is reduced, to effectively prevent the medicament in the medicament passing member from undergoing a boron neutron capture reaction with neutrons in the environment, and avoid a decrease of the content of an effective boron medicament in the medicament passing member, thereby improving efficiency of medicament supplying in therapy.

## Description

### TECHNICAL FIELD

One aspect of the present invention relates to the medical field, and in particular to a medicament supply apparatus. Another aspect of the present invention relates to the field of ray irradiation, and in particular to a neutron capture therapy system.

### BACKGROUND

With the development of atomic science, a radiotherapy such as cobalt sixty, a linear accelerator, and an electronic beam has become one of the main measures for treatment of cancers. However, a traditional photon or electron therapy is limited by a physical condition of radioactive rays. A large number of normal tissues in a path of a beam are also damaged while tumor cells are killed. In addition, because of different degrees of sensitivity of the tumor cells to the radioactive rays, the traditional radiotherapy usually has a poor treatment effect on a radiation-resistant malignant tumor (such as: glioblastoma multiforme and melanoma).

In order to reduce radiation damage to normal tissues around a tumor, a concept of target treatment in a chemotherapy is applied to the radiotherapy. Radiation sources having a high relative biological effectiveness (RBE) are also actively being developed currently for high-radiation-resistant tumor cells, for example, proton therapy, heavy particle therapy, and neutron capture therapy. The neutron capture therapy combines the foregoing two concepts. For example, by specific aggregation of a boron-containing medicine in tumor cells, in combination with precise neutron beam control, a boron neutron capture therapy provides better options for cancer treatment than the traditional radiotherapy.

By the specific aggregation of the boron-containing medicine in human tumor cells, in combination with precisely controllable neutron beam irradiation, the boron neutron capture therapy (BNCT) provides the better options for cancer treatment than the traditional radiotherapy. In the boron neutron capture therapy, first, the boron-containing medicine is injected into a patient. The medicine has a strong affinity with tumor cells, and can be selectively accumulated in the tumor cells. Then, a neutron beam is irradiated to a tumor site of the patient. When captured by ¹⁰B in the tumor cells, neutrons can undergo fission to generate α particles and ⁷Li particles and release a ray with high killability. This ray has a short range that is only equal to a length of one tumor cell, so that the tumor cells can be precisely killed without damaging surrounding normal cells as much as possible. Since a concentration of the boron-containing medicine in the body of the patient decreases with exposure to the neutron beam in the treatment process, in order to maintain a suitable boron concentration value for treatment in the body of the patient, it is hoped to continuously inject the boron-containing medicine to the patient before and during the treatment.

### SUMMARY

In view of this, to solve the above problem, one aspect of the present invention provides a medicament supply apparatus applied to a neutron capture therapy system. The medicament supply apparatus includes: a medicament passing member, configured to supply a medicament to a to-be-irradiated body; and a containing mechanism, configured to contain the medicament passing member, the containing mechanism including a first container arranged on a partition wall. By designing the containing mechanism, the medicament passing member outside a shielding door can bypass the shielding door and directly enter an irradiation room, without affecting normal closing of the shielding door. Meanwhile, in a process of moving the to-be-irradiated body from a medicament control room to the irradiation room, medicament supplying does not need to be stopped, thereby achieving a purpose of continuously supplying the medicament to the to-be-irradiated body.

In an embodiment, the partition wall includes a first wall and a second wall formed with a slot body, and the first wall and the second wall are not in a same plane. A containing mechanism is arranged on different walls. The containing mechanism is arranged according to shapes of the walls, so that it is convenient to contain and maintain the medicament passing member therein.

In an embodiment, the first wall includes a first wall portion; the second wall includes a second wall portion formed with a first slot; the first wall portion is connected to the second wall portion; the first container includes a first containing portion arranged at the first wall portion, and a second containing portion arranged in the first slot; and one end of the first containing portion is connected to one end of the second containing portion. When at least a portion of the shielding door is arranged on one side of the partition wall, by the arrangement of the containing portion, the medicament passing member outside the shielding door may bypass the shielding door and enter the irradiation room, thereby implementing continuous medicament supplying.

In an embodiment, the second wall further includes a third wall portion formed with a second slot, and a fourth wall portion formed with a third slot; the second wall portion, the third wall portion, and the fourth wall portion are connected to form a U-shaped structure; the first container further includes a third containing portion arranged in the second slot, and a fourth containing portion arranged in the third slot; another end of the second containing portion is connected to one end of the third containing portion; and another end of the third containing portion is connected to one end of the fourth containing portion. When at least a portion of the shielding door is arranged on the partition wall, by the arrangement of the containing portion, the medicament passing member outside the shielding door may bypass the shielding door and enter the irradiation room, thereby implementing continuous medicament supplying.

In an embodiment, the first container further includes a stop part; and the stop part allows the medicament passing member to be placed and prevents the medicament passing member from leaving the first container without an external force. By the arrangement of the stop part, the medicament passing member is maintained inside the first container, to avoid that the medicament passing member is pressed by the shielding door in a medicament supplying process, thereby ensuring efficiency of medicament supplying.

In an embodiment, the stop part is correspondingly arranged on a corresponding containing portion, and at least a portion of the stop part extends in a direction from one side edge to another side edge of the containing portion. The stop part is arranged at an edge of the slot, and is closer to an outer side of the containing mechanism than the medicament passing member, so that the medicament passing member is arranged on an inner side of the containing mechanism, thereby achieving a purpose of continuously supplying the medicament to the to-be-irradiated body.

In an embodiment, the containing mechanism includes a second container arranged on a floor. By the arrangement of the second container, exposure of the medicament passing member in the irradiation room to a neutron irradiation environment is reduced, to effectively prevent the medicament in the medicament passing member from undergoing boron neutron capture reaction with neutrons in the environment, and reduce loss of the content of an effective boron medicament in a medicament transmission process, thereby improving efficiency of medicament supplying in therapy.

In an embodiment, the second container includes a linear containing portion and a linear covering portion configured to cover the linear containing portion. The arrangement of the containing portion and the shielding portion facilitates the placement of the medicament passing member, and reduces the exposure of the medicament passing member in the irradiation room to the neutron irradiation environment, thereby improving efficiency of medicament supplying in therapy.

In an embodiment, the second container further includes a curved containing portion; the curved containing portion is connected to the linear containing portion; and the second container further includes a curved covering portion configured to cover the curved containing portion. The arrangement of the curved containing portion improves applicability of the second container, so as to contain the medicament passing member in irradiation rooms with different layouts.

In an embodiment, the linear covering portion and the curved covering portion are made of neutron shielding materials. The neuron shielding materials prevent the medicament in the medicament passing member from undergoing a boron neutron capture reaction with neutrons in an environment, thereby avoiding a decrease in the content of an effective boron medicament in the medicament passing member.

In an embodiment, the medicament supply apparatus further includes a medicament control member and a medicament storage member; the medicament control member acts on the medicament passing member to control medicament supplying to the to-be-irradiated body; the medicament storage member stores a medicament required by the to-be-irradiated body and is connected to the medicament passing member; and the medicament control member and the medicament storage member are physically spatially separated from the second container. The medicament control member and the medicament storage member are physically spatially separated from the second container, so that it avoids the impact of neutron radiation rays in the irradiation room on the medicament storage member and the medicament control member. For example, an electronic element in the medicament control member cannot work normally or reacts with the medicament contained in the medicament storage member.

Another aspect of the present invention provides a neutron capture therapy system, including a charged particle beam generation part, configured to generate a charged particle beam; a neutron beam generation part, configured to generate a therapeutic neutron beam; a beam transmission part, configured to transmit the charged particle beam to the neutron beam generation part; an irradiation room, configured to perform neutron beam radiotherapy on an to-be-irradiated body; a medicament control room, configured to control medicament supplying to the to-be-irradiated body; and the above medicament supply apparatus, configured to supply a medicament to the to-be-irradiated body. By the arrangement of the medicament supply apparatus in the neutron capture therapy system, continuous medicament supplying to the to-be-irradiated body before and during neutron irradiation treatment, so that a proper boron concentration value for treatment is maintained in the body of the to-be-irradiated body, and treatment efficiency is improved.

### BRIEF DESCRIPTION OF THE DRAWINGS

FIG. 1 is a schematic diagram of a planar layout design of a neutron capture therapy system according to an embodiment of the present invention.
FIG. 2 is a schematic diagram of positions of a partition wall and a shielding door according to an embodiment of the present invention, at least a portion of the shielding door being arranged on one side of the partition wall.
FIG. 3 is a schematic diagram of positions of a partition wall and a shielding door according to an embodiment of the present invention, at least a portion of the shielding door being arranged in the partition wall.
FIG. 4 is a schematic diagram of a position of a containing mechanism when at least a portion of a shielding door is arranged on one side of a partition wall according to an embodiment of the present invention.
FIG. 5 is a schematic diagram of a position of a containing mechanism when at least a portion of a shielding door is contained in a partition wall according to an embodiment of the present invention.
FIG. 6 is a schematic structural diagram of a first wall and a second wall when at least a portion of a shielding door is arranged on one side of a partition wall according to an embodiment of the present invention.
FIG. 7 is a schematic structural diagram of a first wall and a second wall when at least a portion of a shielding door is contained in a partition wall according to an embodiment of the present invention.
FIG. 8 is an axonometric drawing of a first container when at least a portion of a shielding door is arranged on one side of a partition wall according to an embodiment of the present invention.
FIG. 9 is a top view of a first container when at least a portion of a shielding door is arranged on one side of a partition wall according to an embodiment of the present invention.
FIG. 10 is an axonometric drawing of a first container when at least a portion of a shielding door is contained in a partition wall according to an embodiment of the present invention.
FIG. 11 is a top view of a first container when at least a portion of a shielding door is contained in a partition wall according to an embodiment of the present invention.
FIG. 12 is a front view of a first container when at least a portion of a shielding door is contained in a partition wall according to an embodiment of the present invention.
FIG. 13 is a top view of a second container according to an embodiment of the present invention.
FIG. 14 is a cross-sectional view of a second container according to an embodiment of the present invention.
FIG. 15 is a top view of a linear containing portion of a second container according to an embodiment of the present invention.
FIG. 16 is an axonometric drawing of a linear containing portion of a second container according to an embodiment of the present invention.
FIG. 17 is a top view of a curved containing portion of a second container according to an embodiment of the present invention.
FIG. 18 is an axonometric drawing of a curved containing portion of a second container according to an embodiment of the present invention.

### Descriptions of reference numerals:

100: neutron capture therapy system; 101: (first) irradiation room; 101': second irradiation room; 102: accelerator room; 103: beam transmission room; 104: (medicament) control room; 10: beam generation apparatus; 11: charged particle beam generation part; 111: ion source; 112: accelerator; 12: beam transmission part; 121: beam direction switching assembly; 13: (first) neutron beam generation part; 13': second neutron beam generation part; 14: auxiliary device; 20: (first) treatment table; 20': second treatment table; 30: beam collector; 40: medicament supply apparatus; 41: medicament storage member; 42: medicament control member; 43: medicament passing member; 44: containing mechanism; 441: first container; 4411: first containing portion; 4412: second containing portion; 4413: third containing portion; 4413a: convex edge part; 4414: fourth containing portion; 4415: fifth containing portion; 4416: sixth containing portion; 4417: stop part; 442: second container; 4421: linear containing portion; 4422: curved containing portion; 4423: linear covering portion; 4424: curved covering portion; 200: to-be-irradiated body; W1: partition wall; W2: partition wall; W21: first wall; W211: first wall portion; W212: fifth wall portion; W22: second wall; W221: second wall portion; W222: third wall portion; W223: fourth wall portion; W3: floor; G1: first slot; G2: second slot; G3: third slot; D1: shielding door; and 4425: boss.

### DETAILED DESCRIPTION

In order to make the aforementioned objectives, features, and advantages of the present invention more comprehensible, specific implementations of the present invention are described in detail below in conjunction with the accompanying drawings. In the following description, numerous specific details are set forth in order to provide a thorough understanding of the present invention. The present invention may, however, be embodied in many forms different from that described here. A person skilled in the art can make similar improvements without departing from the connotation of the present invention. Therefore, the present invention is not limited by the specific embodiments disclosed below.

In the descriptions of the present invention, it should be understood that orientations or positional relationships indicated by the technical terms "center", "longitudinal", "transverse", "length", "width", "thickness", "upper", "lower", "front", "rear", "left", "right", "vertical", "horizontal", "top", "bottom", "inner", "outer", "clockwise", "anticlockwise", "axial", "radial", "circumferential", and the like are orientations or positional relationships as shown in the drawings, and are only for the purpose of facilitating and simplifying the descriptions of the present invention instead of indicating or implying that devices or elements indicated must have particular orientations, and be constructed and operated in the particular orientations, so that these terms are not construed as limiting the present invention.

In addition, terms "first" and "second" are used merely for the purpose of description, and shall not be construed as indicating or implying relative importance or implying a quantity of indicated technical features. Therefore, a feature restricted by "first" or "second" may explicitly indicate or implicitly include at least one of such features. In the description of the present invention, "plurality" means at least two, for example, two and three, unless explicitly specified otherwise.

In the present invention, unless otherwise explicitly specified and limited, the terms "mount", "connect", "connection", and "fix" should be understood in a broad sense. For example, a connection may be a fixed connection, a detachable connection, or an integral connection; or the connection may be a mechanical connection or an electrical connection; or the connection may be a direct connection, an indirect connection through an intermediary, or internal communication between two elements or mutual action relationship between two elements, unless otherwise specified explicitly. Persons of ordinary skill in the art may understand the specific meanings of the foregoing terms in the present invention according to specific situations.

In the present invention, unless otherwise explicitly stipulated and restricted, that a first feature is "on" or "below" a second feature may include that the first and second features are in direct contact, or may include that the first and second features are in indirect contact using an intermediate. In addition, that the first feature is "above", "over", or "on" the second feature may indicate that the first feature is directly above or obliquely above the second feature, or may merely indicate that the horizontal position of the first feature is higher than that of the second feature. That the first feature is "below", "under", and "beneath" the second feature may be that the first feature is right below the second feature or at an inclined bottom of the second feature, or may merely indicate that the horizontal position of the first feature is lower than that of the second feature.

It should be noted that, when an element is referred to as being "fixed to" or "arranged at" another element, the element can be directly on another component or there can be a centered element. When an element is considered to be "connected" to another element, the element can be directly connected to another element or there may be a centered element. The terms "vertical", "horizontal", "up", "down", "left", "right", and similar expressions used herein are for illustrative purposes only and do not necessarily represent the only implementation.

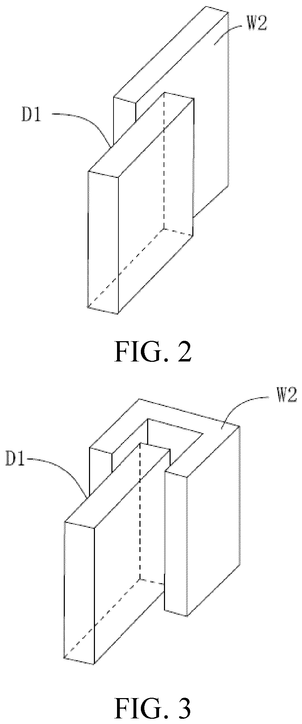

It should be noted that, although structures of elements are slightly different in different embodiments, for ease of description, components having same or similar functions in different embodiments use the same reference numerals. Herein, the same reference numerals are not used to explain exactly the same structures. For example, for a partition wall W2 shown below and in the accompanying drawings, partition walls in FIG. 2 and FIG. 3 have slightly different structures. For ease of description, the same reference numeral is used in FIG. 2 and FIG. 3.

As an effective measure for treating cancers, neutron capture therapy has been increasingly widely applied in recent years. Boron neutron capture therapy is the most common. Neurons for the boron neutron capture therapy may be supplied by a nuclear reactor or an accelerator. The embodiments of the present invention take accelerator boron neutron capture therapy as an example. Basic assemblies of the accelerator boron neutron capture therapy usually include an accelerator for accelerating charged particles (such as protons or deuterons), a target, a heat removing system, and a beam shaper. Accelerated charged particles acts on a metal target to generate neurons. An appropriate nuclear reaction is selected according to characteristics such as a desired neutron production rate and energy, energy and current of accelerated charged particles that can be provided, and physicochemical properties of the metal target. Nuclear reactions that are usually discussed are as follows: ⁷Li (p, n)⁷Be and ⁹Be (p, n)⁹B, both of which are heat absorption reactions. Energy thresholds of the two nuclear reactions are respectively 1.881 MeV and 2.055 MeV. Since an ideal neutron source for the boron neutron capture therapy is a super-thermal neutron with an energy level of keV. Theoretically, if a proton with energy that is slightly greater than the thresholds is used to bombard a metal lithium target material, a relatively low-energy neutron may be generated, and the neutron can be used clinically without much slowdown treatment. However, two target materials, namely, metal lithium (Li) and a metal beryllium (Be), do not have a large action cross section with a proton with threshold energy. To generate a large enough neutron flux, a proton with relatively high energy is usually selected to initiate a nuclear reaction.

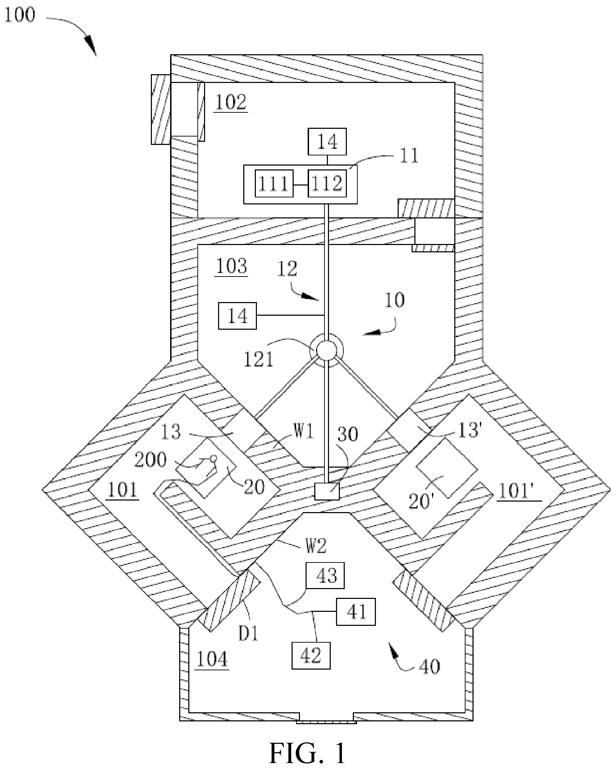

Referring to FIG. 1, FIG. 1 is a schematic diagram of a planar layout design of a neutron capture therapy system according to an embodiment of the present invention. The neutron capture therapy system in this embodiment is preferably a boron neutron capture therapy system 100. In a process of the boron neutron capture therapy, a to-be-irradiated body 200 injected with a medicament, such as a B-10-containing medicine, is irradiated with a neutron beam. A boron(B-10)-containing medicine has a very strong affinity with tumor cells, and may be selectively accumulated inside the tumor cells. By using the characteristic that the boron(B-10)-containing medicine has a high capture cross section for thermal neutrons, when the neutrons are captured by ¹⁰B in the tumor cells, the neutrons may undergo fission to generate α particles and ⁷Li particles. Average energy of the two types of particles is approximately 2.33 MeV, featuring high linear energy transfer and short range. The linear energy transfer and range of the α particles are respectively 150 keV/um and 8 um, and the linear energy transfer and range of the ⁷Li particles are respectively 175 keV/um and 5 um. A total range of the two types of particles is approximately equivalent to a size of a cell. Radiation damage to a living body is limited at a cell level, so that the tumor cells can be precisely killed without damaging surrounding normal cells as much as possible.

The boron neutron capture therapy system 100 includes a beam generation apparatus 10 and a (first) treatment table 20. The beam generation apparatus 10 includes a charged particle beam generation part 11, a beam transmission part 12, and a (first) neutron beam generation part 13. The charged particle beam generation part 11 generates a charged particle beam P such as a proton beam. The beam transmission part 12 transmits the charged particle beam P to the neutron beam generation part 13. The neutron beam generation part 13 generates a therapeutic neutron beam N and irradiates the to-be-irradiated body 200 on the treatment table 20. The boron neutron capture therapy system 100 further includes an auxiliary device 14. The auxiliary device 14 may include any auxiliary device for providing a precondition for operation of the charged particle beam generation part 11, the beam transmission part 12, and the subunit generation portion 13.

Referring to FIG. 1 again, the entire boron neutron capture therapy system 100 is contained in a building constructed with concrete. Specifically, the boron neutron capture therapy system 100 includes a (first) irradiation room 101, an accelerator room 102, and a beam transmission room 103. The to-be-irradiated body 200 on the treatment table 20 is treated by irradiation of the neutron beam N in the irradiation room 101. The accelerator room 102 at least partially contains the charged particle beam generation part 11. The charged particle beam generation part 11 including an ion source 111 and an accelerator 112. The ion source 111 is configured to generate charged particles such as H-, a proton, or a deuteron. The accelerator 112 accelerates the charged particles generated by the ion source 111 to obtain the charged particle beam P, such as a proton beam, with desired energy. The accelerator 112 may be a linear accelerator, a cyclotron, a synchrotron, or a synchro-cyclotron. The beam transmission room 103 at least partially contains the beam transmission part 12. At least a portion of the neutron beam generation part 13 is contained in a partition wall W1 for the irradiation room 101 and the beam transmission room 103, and at least a portion of the auxiliary device 14 is arranged in the accelerator room 102 or the beam transmission room 103.

The boron neutron capture therapy system 100 may further include a second irradiation room 101'. The beam generation apparatus 10 further includes a second neutron beam generation part 13' corresponding to the second irradiation room 101'. The beam transmission part 12 includes a beam direction switching assembly 121. The beam transmission part 12 selectively transmits the charged particle beam P generated by the charged particle beam generation part 11 to the first neutron beam generation part 13 or the second neutron beam generation part 13' through the beam direction switching assembly 121, thereby emitting a beam into the first irradiation room 101 or the second irradiation room 101'. It should be understood that the neutron beam N irradiated to the second irradiation room 101' may be as a neutron beam N for treating another to-be-irradiated body on the second treatment table 20' in the second irradiation room 101', or may be applied to sample detection, or the like. The present invention does not impose a limitation on this.

It should be understood that the beam generation apparatus 10 may further have another construction. For example, when there is a third irradiation room, a third neutron beam generation part may be added to correspond to the third irradiation room. A quantity of the neutron beam generation parts corresponds to a quantity of the irradiation rooms. This embodiment of the present invention does not impose a specific limitation on the quantity of the neutron beam generation parts. Arranging one charged particle beam generation part for transmission to the neutron beam generation parts can effectively reduce system costs. It can be understood that the beam generation apparatus may further include a plurality of charged particle beam generation parts for transmission to the neutron beam generation parts, and a plurality of neutron beams may be simultaneously generated in a plurality of irradiation rooms for irradiation.

In an embodiment of the present invention, the beam direction switching assembly 121 includes a deflection magnet (not shown in the figure) that deflects a direction of the charged particle beam P. If the deflection magnet corresponding to the first irradiation room 101 is turned on, a beam is introduced into the first irradiation room 101. The present invention does not impose a specific limitation on this. The boron neutron capture therapy system 100 may further include a beam collector 30 which collects a beam when the beam is not required, confirms outputting of the charged particle beam P before treatment, or the like. The beam direction switching assembly 121 can enable the charged particle beam P to leave a regular track and be guided to the beam collector.

The boron neutron capture therapy system 100 may further include a preparing room (not shown in the figure), a control room 104, and another space (not shown in the figure) for adjuvant treatment. A preparation room may be configured for each irradiation room for preparation work such as fixing of a to-be-irradiated body to a treatment table before radiotherapy, simulated placement of the to-be-irradiated body, and simulation of a treatment plan. The control room 104 is configured to control the accelerator, the beam transmission part, the treatment table, and the like, and control and manage an entire irradiation process. An administrator may further simultaneously monitor a plurality of irradiation rooms in the control room. Only one configuration manner of the control room is shown in the figure. It can be understood that the control room may further have another configuration.

Since a medicament needs to be continuously supplied in the boron neutron capture therapy, the boron neutron capture therapy system 100 further includes a medicament control room (which is the control room 104 in this embodiment) and a medicament supply apparatus 40. The medicament supply apparatus 40 is configured to supply a boron(B-10)-containing medicine to the to-be-irradiated body 200 before and during the neutron beam irradiation treatment. By the arrangement of the medicament supply apparatus 40 in the neutron capture therapy system 100, continuous medicament supplying to the to-be-irradiated body 200 before and during neutron irradiation treatment, so that a proper boron concentration value for treatment is maintained in the body of the to-be-irradiated body 200, and treatment efficiency is improved. The medicament supply apparatus 40 includes a medicament storage member 41, a medicament control member 42, and a medicament passing member 43. The medicament storage member 41 is configured to store a medicament required by the to-be-irradiated body 200. The medicament control member 42 is configured to control medicament supplying to the to-be-irradiated body 200. The medicament passing member 43 is configured to supply the medicament to the to-be-irradiated body 200. The medicament storage member 41 and the medicament control member 42 may be arranged in the medicament control room 104 and control the supplying of the boron(B-10)-containing medicine to the to-be-irradiated body 200 in the medicament control room 104, thereby avoiding the impact of neutron radiation rays in the irradiation room 101 on the medicament storage member 41 and the medicament control member 42. For example, an electronic element in the medicament control member 42 cannot work normally or reacts with the boron(B-10)-containing medicine in the medicament storage member 41. The medicament passing member 43 is connected to the medicament storage member 41, and supplies the boron(B-10)-containing medicine to the to-be-irradiated body 200 through the medicament control member 42. The medicament storage member 41 may consist of an infusion bag, an infusion bottle, or the like. The medicament control member 42 may control flowing of the boron(B-10)-containing medicine in the medicament passing member 43. For example, an infusion pump is used to provide flowing power of the medicament (the boron(B-10)-containing medicine), and a flow rate of the medicament may be controlled. The medicament control member 42 may further have functions of detection, alarm, and the like. The medicament passing member 43 may be a disposable infusion pipe or the like, for example, including a syringe needle inserted into the to-be-irradiated body 200, a syringe needle protection sleeve, a hose, a connector connected to the medicament storage member 41, and the like. The medicament passing member 43 may alternatively be at least partially made of a neutron shielding material, for example, the syringe needle or the hose that is exposed in the irradiation room 101. This can reduce impact of the neutron radiation rays in the irradiation room 101 on the born- (B-10)-containing medicine in the medicament passing member 43. The figure merely shows the apparatus for supplying the boron medicament to the to-be-irradiated body 200 in the first irradiation room 101. It can be understood that the same medicament supply apparatus 40 may alternatively be used to supply a boron medicine to a to-be-irradiated body in another irradiation room.

Referring to FIG. 2 and FIG. 3, FIG. 2 and FIG. 3 show schematic diagrams of positions of a partition wall and a shielding door according to an embodiment of the present invention. FIG. 2 shows that at least a portion of the shielding door is arranged on one side of the partition wall. For example, at least a portion of the shielding door is located on one side of the partition wall away from an irradiation room. FIG. 3 shows that at least a portion of the shielding door is contained in the partition wall. For example, in a closed state of the shielding door, at least a portion of the shielding door is contained in a space formed by the partition wall. As shown in FIG. 1, the partition wall W2 and a shielding door D1 for separating the irradiation room 101 from the medicament control room 104 are arranged between the medicament control room 104 and the irradiation room 101. Entering and leaving the irradiation room 101 may be performed through the shielding door D1. The partition wall W2 and the shielding door D1 jointly play a role of shielding the neutron radiation rays in the irradiation room 101 during neutron beam irradiation. The shielding door D1 includes a radiation ray shielding component such as a lead plate, a driving mechanism, and a guide mechanism. When the shielding door D1 is in the closed state, the shielding door D1 and the partition wall W2 jointly implement complete closure of the irradiation room 101, to prevent the neutron radiation rays from being irradiated out of the irradiation room 101. It can be understood that provided that the shielding door D1 can play the role of shielding the neutron radiation rays in the irradiation room 101, the shielding door D1 and the partition wall W2 may have another positional relationship according to a structural feature of the shielding door D1.

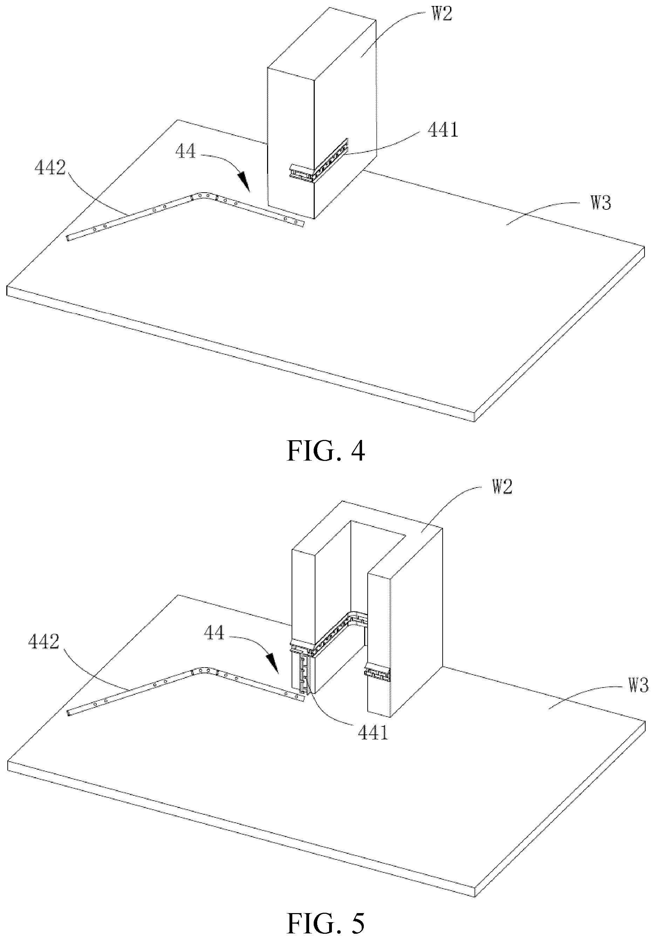

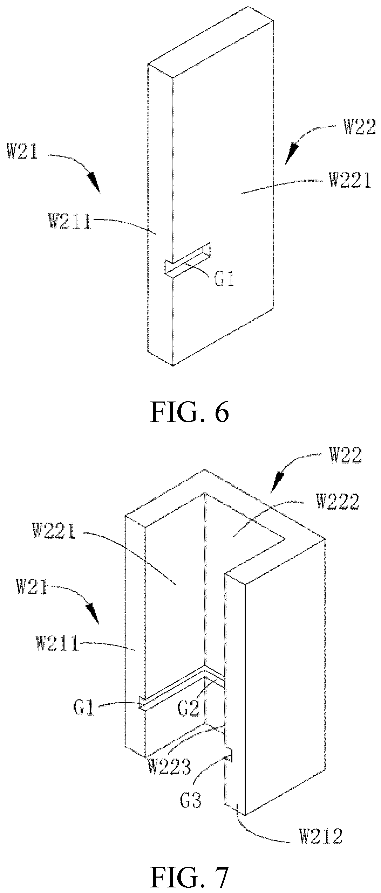

Referring to FIG. 4 and FIG. 5, FIG. 4 and FIG. 5 respectively show schematic diagrams of positions of a containing mechanism when at least a portion of a shielding door is arranged on one side of a partition wall according to an embodiment of the present invention and when at least a portion of a shielding door is contained in a partition wall according to another embodiment of the present invention. As shown in FIG. 1, the medicament supply apparatus 40 further includes a containing mechanism 44 that is arranged between the medicament control room 104 and the irradiation room 101 and in the irradiation room 101, and that is configured to contain the medicament passing member 43, so that the medicament passing member 43 that is configured to supply the boron(B-10)-containing medicine and that is outside the shielding door D1 may bypass the shielding door D1 and directly enter the irradiation room 101. Further, the containing mechanism 44 includes a first container 441 arranged on the partition wall W2. By designing the first container 441, the medicament passing member 43 outside the shielding door W2 can bypass the shielding door D1 and directly enter the irradiation room 101, without affecting normal closing of the shielding door D1. Meanwhile, in a process of moving the to-be-irradiated body 200 from the medicament control room 104 to the irradiation room 101, medicament supplying does not need to be stopped, thereby achieving a purpose of continuously supplying the medicament to the to-be-irradiated body 200. In a feasible embodiment, the containing mechanism 44 further includes a second container 442 arranged on a floor W3. By the arrangement of the second container 442, exposure of the medicament passing member 43 in the irradiation room 101 to a neutron irradiation environment is reduced, to effectively prevent the medicament in the medicament passing member 43 from undergoing boron neutron capture reaction with neutrons in the environment, and reduce loss of the content of an effective boron medicament in a medicament transmission process, thereby improving efficiency of medicament supplying in therapy. As shown in FIG. 1, the medicament control member 42 and the medicament storage member 41 are physically spatially separated from the second container 441, so that it avoids the impact of the neutron radiation rays in the irradiation room 101 on the medicament storage member 41 and the medicament control member 42. Referring to FIG. 6 and FIG. 7, FIG. 6 and FIG. 7 respectively show schematic structural diagrams of a first wall when at least a portion of a shielding door is arranged on one side of a partition wall according to an embodiment of the present invention and when at least a portion of the shielding door is contained in a partition wall according to another embodiment of the present invention. The partition wall W2 includes a first wall W21 and a second wall W22 formed with a slot body, and the first wall W21 and the second wall W22 are not in the same plane. In this embodiment, the second wall W22 is closer to the shielding door D1 than the first wall W21. The second wall W22 abuts against the shielding door D1, thereby ensuring a shielding effect of the shielding door D1. Referring to FIG. 2 and FIG. 6, when at least a portion of the shielding door D1 is arranged on one side of the partition wall W2, the first wall W21 includes a first wall portion W211; the second wall W22 includes a second wall portion W221 formed with a first slot G1; and the first wall portion W211 and the second wall portion W221 are not in the same plane. In this embodiment, the first wall portion W211 is perpendicular to the second wall portion W221. Referring to FIG. 2 and FIG. 7, when at least a portion of the shielding door D1 is contained in the partition wall W2, the second wall W22 arranged around an edge of the shielding door D1 further includes a third wall portion W222 formed with a second slot G2, and a fourth wall portion W223 formed with a third slot G3. The second wall portion W221, the third wall portion W222, and the fourth wall portion W223 are connected to form a U-shaped structure. Correspondingly, the first slot G1, the second slot G2, and the third slot G3 are also connected to form a U-shaped structure. In this embodiment, the second wall portion W221 is parallel to the fourth wall portion W223. The third wall portion W222 is parallel to the first wall portion W211. The third wall portion W222 is perpendicular to both the second wall portion W221 and the fourth wall portion W223. The second wall portion W221, the third wall portion W222, and the fourth wall portion W223 are connected to form a containing cavity that may partially contain the shielding door D1. In a feasible embodiment, the first wall W21 further includes a fifth wall portion W212 connected to the fourth wall portion W223, and the fourth wall portion W223 and the fifth wall portion W212 are not in the same plane. In this embodiment, the fourth wall portion W223 is perpendicular to the fifth wall portion W212, and the fifth wall portion W212 is parallel to the first wall portion W211. It can be understood that provided that the partition wall W2 is combined with the shielding door D1, the partition wall W2 may further have another formation manner of wall portions, and a positional relationship between the wall portions is not limited.

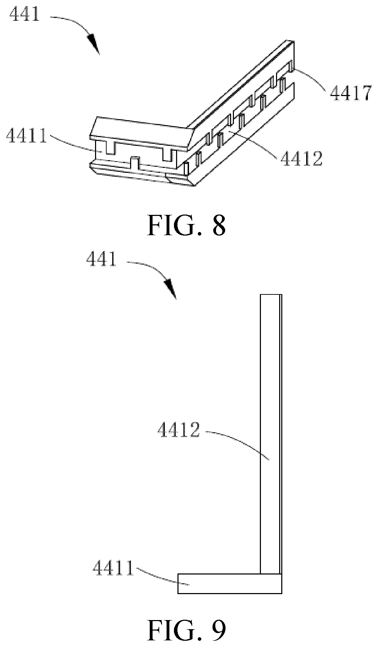

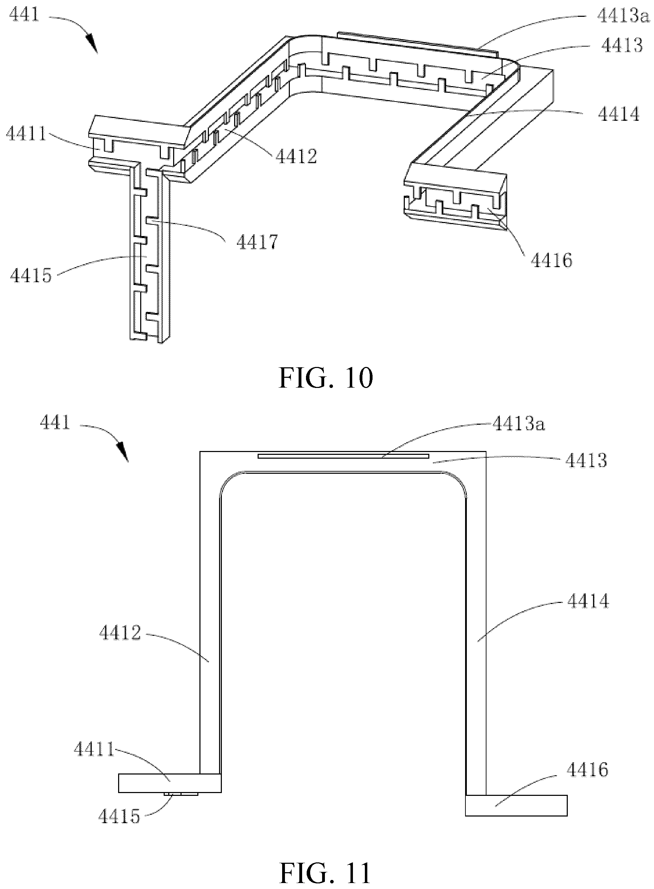

Referring to FIG. 8 to FIG. 12, FIG. 8 to FIG. 12 respectively show schematic diagrams of a first container when at least a portion of a shielding door is arranged on one side of a partition wall according to an embodiment of the present invention and when at least a portion of a shielding door is contained in a partition wall according to another embodiment of the present invention. Referring to FIG. 8 and FIG. 9, as shown in FIG. 2 to FIG. 7, when at least a portion of the shielding door D1 is arranged on one side of the partition wall W2, the first container 441 is configured to contain and maintain the medicament passing member 43. The first container 441 includes a first containing portion 4411 arranged at the first wall portion W211, and a second containing portion 4412 arranged in the first slot G1. One end of the first containing portion 4411 is connected to one end of the second containing portion 4412. It can be understood that an extension direction of the first containing portion 4411 is not limited provided that the first containing portion 4411 can be connected to the second containing portion 4412 without affecting the operation of the shielding door D1. Preferably, the first containing portion 4411 extends towards the second container 442. By using a flexible material, according to the structural design of the first containing portion 4411 and the second containing portion 4412, the medicament passing member 43 is bent or suddenly changed to adapt to the structural design or is formed into a shape matched with a shape of the structural design. For example, if a sudden change occurs at a joint between the first containing portion 4411 and the second containing portion 4412, the medicament passing member 43 is also suddenly changed at the joint, but the medicament is still allowed to pass. For another example, if the first containing portion 4411 and the second containing portion 4412 are combined to form an L shape, the medicament passing member 43 is also formed in a shape matching manner into an L shape or a shape similar to the L shape, but the medicament is still allowed to pass. Referring to FIG. 10 to FIG. 12, as shown in FIG. 2 FIG. 7, when at least a portion of the shielding door D1 is contained in the partition wall W2, the first container 441 further includes a third containing portion 4413 arranged in the second slot G2, and a fourth containing portion 4414 arranged in the third slot G3. One end of the second containing portion 4412 is connected to one end of the third containing portion 4413, and another end of the third containing portion 4413 is connected to one end of the fourth containing portion 4414. In a feasible embodiment, the first container 441 further includes a fifth containing portion 4415 arranged at the first wall portion W211, and a sixth containing portion 4416 arranged at the fifth wall portion W212. The fifth containing portion 4415 extends in a direction towards the second container 442. One end of the fifth containing portion 4415 is connected to another end of the first containing portion 4411. Another end of the fourth containing portion 4414 is connected to one end of the sixth containing portion 4416. Similar to the above description, according to the structural design of the containing portions, the medicament passing member 43 is bent or suddenly changed to adapt to the structural design or is formed into a shape matched with the shape of the structural design, and allows the medicament to pass through. In addition, when the shielding door D1 is in the closed state, since a preset distance exists between the first container 441 that supports the medicament passing member 43 and the shielding door D1, for example, since a portion of the first container 441 is located in the partition wall W2, the first container 441 does not interfere with the operation of the shielding door D1, thereby avoiding blockage of a medicament flowing path caused by the medicament passing member 43 being pressed by the shielding door D1.

Referring to FIG. 10 to FIG. 12 again, in this embodiment, the first wall portion W211, the third wall portion W222, and the fifth wall portion W212 are parallel to a thickness direction of the shielding door D1, and the second wall portion W221 and the fourth wall portion W223 are perpendicular to the thickness direction of the shielding door D1. Outer sides of the second containing portion 4412, the third containing portion 4413, and the fourth containing portion 4414 are respectively flush with surfaces of the second wall portion W221, the third wall portion W222, and the fourth wall portion W223. The first containing portion 4411 and the fifth containing portion 4415 protrude out of a surface of the first wall portion W211, and the sixth containing portion 4416 protrudes out of a surface of the fifth wall portion W212. The first containing portion 4414, the fifth containing portion 4415, and the sixth containing portion 4416 are fixed on the first wall portion W211 and the fifth wall portion W212 by gluing. It can be understood that the first containing portion 4411, the fifth containing portion 4415, and the sixth containing portion 4416 can be fixed to the first wall portion W211 and the fifth wall portion W212 in either a movable connection manner or a rigid connection manner, for example, by pressing fasteners, using screwed connection, using bolted connection, using snap-in connection, or using hinged connection. Alternatively, the first containing portion 4414, the fifth containing portion 4415, and the sixth containing portion 4416 may be respectively contained by slotting the first wall portion W211 and the fifth wall portion W212. In this embodiment, the first containing portion 4411 and the sixth containing portion 4416 are parallel to each other and extend in opposite directions. The fifth containing portion 4415 is perpendicular to the first containing portion 4411, and extends in a direction towards the second container 442. The fifth containing portion 4415 determines an overall height of the first container 441. Preferably, the overall height of the first container 441 is below the medicament storage member 41, to facilitate medicament supplying. It can be understood that the fifth containing portion 4415 may be in any direction, provided that operation of the shielding door D1 is not affected. Adding the fifth containing portion 4415 arranged in the irradiation room 101 and the sixth containing portion 4416 arranged in the medicament control room 104 are added can better implement that the medicament passing member 43 inside the control room 104 can bypass the shielding door D1 and enter the irradiation room 101, and a better protection effect can be achieved on the medicament passing member 43.

Referring to FIG. 10 to FIG. 12 again, in this embodiment, one side of the second containing portion 4412, one side of the third containing portion 4413, and one side of the fourth containing portion 4414 of the first container 441 are configured to contain the medicament passing member 43, and their another sides are respectively configured to cooperate with the first slot G1, the second slot G2, and the third slot G3 to partially mount the second containing portion 4412, the third containing portion 4413, and the fourth containing portion 4414 in the first slot G1, the second slot G2, and the third slot G3. On the sides for containing the medicament passing member 43, the second containing portion 4412, the third containing portion 4413, and the fourth containing portion 4414 are further provided with straight upper edge portions and straight lower edge portions. The upper edge portions and the lower edge portions are configured to further fixedly cooperate with the first slot G1, the second slot G2, and the third slot G3, so that the first container 441 is better fixed inside the first wall W2. The upper edge portions and the lower edge portions are of planar structures, so as not to affect the normal operation of the shielding door D1. The first containing portion 4411 and the sixth containing portion 4416 include inclined upper edge portions and inclined lower edge portions, to facilitate the overall design of the first container 441. An upper side of the third containing portion 4413 further includes a convex edge part 4413a arranged on an inner side of the fourth wall portion W223, and extends in a height direction of the fourth wall portion W223. The convex edge part 4413a is different from the upper edge portion of the third containing portion 4413 and is configured for the further fixed cooperation between the first containing portion 441 with the partition wall W2. It can be understood that provided that the first container 441 may be fixed to the first wall (the partition wall W2), the first container 441 may not be provided with the above edge portions. To better contain the medicament passing member 43, an inner diameter of the first container 441 needs to be greater than an outer diameter of the medicament passing member 43. In this embodiment, a cross-sectional shape of a containing cavity of the first container 441 is a square. It can be understood that provided that it can contain the medicament passing member 43, the cross-sectional shape of the containing cavity of the first container 441 may be a circle, a triangle, or the like. The first container 441 is inserted in an operation direction of the shielding door D1 until a portion corresponding to the third containing portion 4413 is abutted with the second slot G2, thereby completing the mounting of the entire first container 441.

Referring to FIG. 8 and FIG. 9, through the first container 441, after the to-be-irradiated body 200 is moved from the medicament control room 104 to the irradiation room 101, the medicament passing member 43 is placed into the second containing portion 4412 and the first containing portion 4411 in sequence. Referring to FIG. 10 to FIG. 12, through the first container 441, after the to-be-irradiated body 200 is moved from the medicament control room 104 to the irradiation room 101, the medicament passing member 43 is placed into the sixth containing portion 4416, the fourth containing portion 4414, the third containing portion 4413, the second containing portion 4412, the first containing portion 4411, and the fifth containing portion 4415 in sequence. The medicament passing member 43 inside the medicament control room 104 may bypass the shielding door D1 and enter the treatment room 100, and medicament supplying to the to-be-irradiated body 200 does not need to be stopped, so that continuous medicament supplying to the to-be-irradiated body 200 is implemented, and treatment efficiency is improved. The first container 441 may further support the medicament passing member 43. While it is convenient for the medicament passing member 43 to pass through, a concrete wall can be isolated to prevent dust or the like from polluting the medicament passing member 43. It can be understood that the first container 441 is arranged along the shielding door D1. Provided that the medicament passing member 43 can pass through and that the shielding door D1 can operate normally, the containing portions of the first container 441 may be correspondingly increased or decreased in the irradiation room and/or the control room, and the quantity of the containing portions and a positional relationship between the containing portions are not limited.

Further, the first container 441 further includes stop parts 4417 which allow the medicament passing member 43 to be placed and prevent the medicament passing member 43 from leaving the first container 441 without an external force. The stop parts 4417 are correspondingly arranged on a corresponding containing portion, and at least some of the stop parts 4417 extend in a direction from one side edge to another side edge of the containing portion. In this embodiment, the stop parts 4417 are of square structures, and are overall distributed in an up-down staggered manner. Partial stop parts extend from one side edge of the containing portion to another side edge, and partial stop parts extend from the another side edge of the containing portion to the one side edge. In a feasible embodiment, the stop parts 4417 are arranged on one side of a containing portion, and all extend from one side edge of the containing portion to another side edge of the containing portion. By the arrangement of the stop parts 4417, the medicament passing member 43 can be placed into the first container 441. Meanwhile, blockage, to the transmission of the medicament, caused by the medicament passing member 43 being pressed by the shielding door D1 because of slippage without an external force in the medicament supplying process is prevented. When the shielding door D1 is in an opened or closed state, a supply state of the medicament in the medicament passing member 43 can be clearly observed through an opening between the stop parts 4417 of the first container 441. It can be understood that provided that the stop parts 4417 can allow the medicament passing member 43 to be placed and prevent the medicament passing member 43 from sliding, the stop parts 4417 may have in various shapes, for example, they may be triangular, polygonal, circular, or the like, and a distance between the stop parts 4417 is not limited.

In an embodiment of the present invention, a material of the first container 441 is polyvinyl chloride (PVC), so that a product irradiated by neurons has no radioactivity or has an extremely low radioactivity, thus reducing generated secondary radiation. It can be understood that another material may alternatively be used, by which, a product irradiated by neurons has no radioactivity, or a product irradiated by neurons has low radioactivity, or radioisotopes generated after irradiation with neurons have a short half-life.

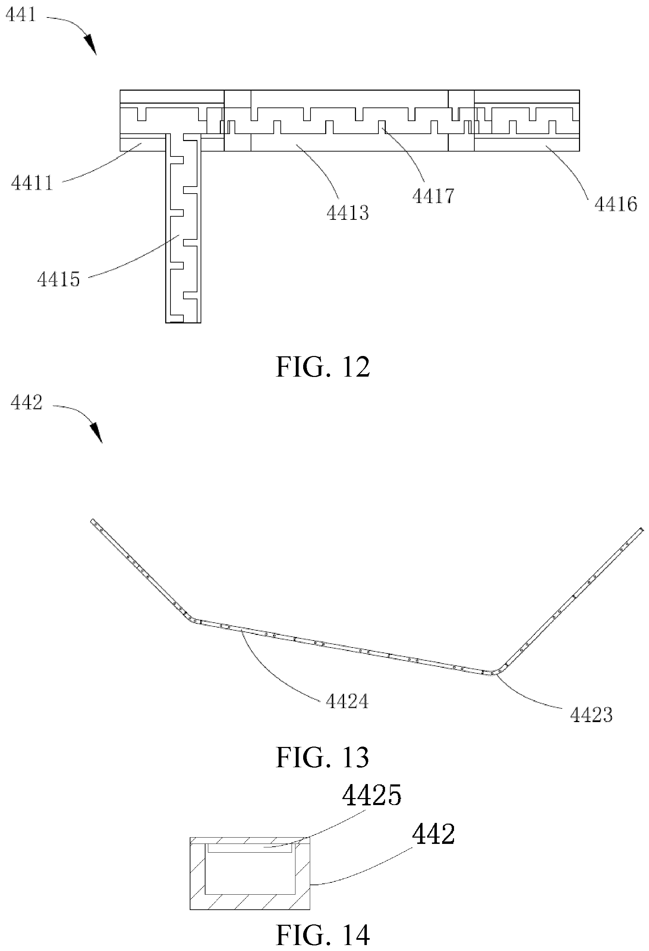

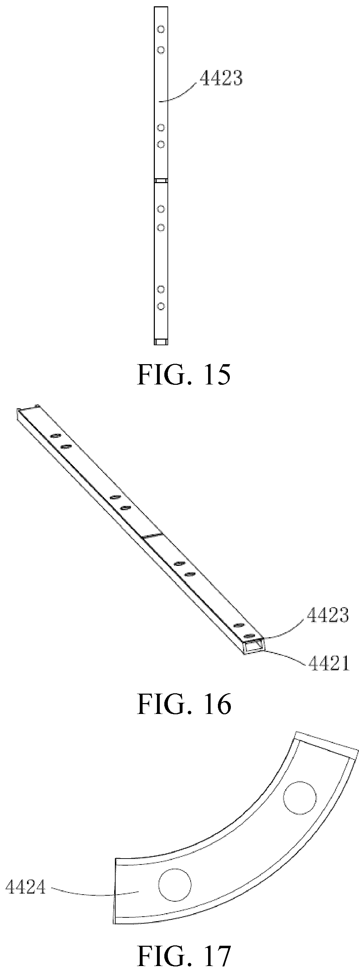

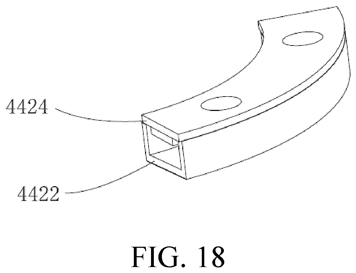

Referring to FIG. 13 and FIG. 14, FIG. 13 and FIG. 14 respectively show a top view and a cross-sectional view of a second container according to an embodiment of the present invention. The second container 442 is arranged in the floor W3 of the irradiation room 101. With reference to FIG. 15 to FIG. 18, the second container 442 includes a linear containing portion 4421 and a curved containing portion 4422 connected to the linear containing portion 4421. The second container 442 further includes a linear covering portion 4423 and a curved covering portion 4424 that are respectively configured to cover the linear containing portion 4421 and the curved containing portion 4422. At least a portion of the first container 441 is physically spatially separated from the second container 442, and one side of the second container 442 is closer to the to-be-irradiated body 200 than the first container 441. In this embodiment, the second container 442 is arranged in the irradiation room 101, and the sixth containing portion 4416 of the first container 441 is arranged in the medicament control room 104, to implement the physically spatial separation. One end of the second container 442 is close to one end of the first container 441, and another end of the second container 442 is close to the treatment table 20 and is closer to the to-be-irradiated body 200 than the first container 441. A length and layout of the second container 442 are determined by a path of the first container 441 from one end inside the irradiation room 101 to the treatment table 20. It can be understood that provided that the second container 442 can reach the treatment table 20, the second container 442 may only have the linear containing portion 4421 and the linear covering portion 4423 or the curved containing portion 4422 and the curved covering portion 4424.

Referring to FIG. 13 and FIG. 15 to FIG. 18, both the linear covering portion 4423 and the curved covering portion 4424 have at least two through holes, so that it is convenient to place and fetch the linear covering portion 4423 and the curved covering portion 4424 and to observe a supply state of the medicament in the medicament passing member 43. The linear covering portion 4423 is in a sectional design, to facilitate use and replacement of the linear covering portion 4423. There is a fit clearance that is as small as possible in the linear covering portion 4423, to reduce the impact of the neutron radiation rays in the irradiation room 101 on the boron(B-10)-containing medicine in the neutron beam irradiation process. Correspondingly, a matching clearance that is as small as possible needs to be reserved between the linear covering portion 4423 and the curved covering portion 4424. It can be understood that the linear covering portion 4423 may alternatively be of an independent integrated structure, and the linear covering portion 4423 and the curved covering portion 4424 may alternatively be of a combined integrated structure.

Referring to FIG. 14, in this embodiment, the linear covering portion 4423 and the curved covering portion 4424 are provided with bosses 4425 having widths that are less than widths of containing cavities of the linear containing portion 4421 and the curved containing portion 4422, so as to facilitate combination with the linear containing portion 4421 and the curved containing portion 4422 and better achieve a shielding effect in the medicament supplying process. Preferably, a surface of the second container 442 is flush with the floor W3 as much as possible, and no significant protrusion is formed, so that the to-be-irradiated body 200 and operation personnel can safely walk in and out of the irradiation room 101. It can be understood that provided that it can contain the medicament passing member 43, a cross-sectional shape of a containing cavity of the second container 442 may be a circular, a square, a V shape, and the like. The second container 442 enables the medicament passing member 43 to be exposed in the irradiation room 101 as little as possible, so that in the neutron beam irradiation process, the impact of the neutron radiation rays in the irradiation room 101 on the boron(B-10)-containing medicine in the medicament passing member 43 is reduced, to improve efficiency of medicament supplying.

In an embodiment of the present invention, a material of the second container 442 is an aluminum Alloy. It can be understood that the second container 442 may alternatively be another material that has a strength, by which, a product irradiated by neurons has no radioactivity, or a product irradiated by neurons has low radioactivity, or radioisotopes generated after irradiation with neurons have a short half-life, such as a carbon fiber composite material or a glass fiber composite material.

A process of supplying the boron(B-10)-containing medicine before and after radiotherapy is as follows: The appropriate medicament passing member 43 is selected before the radiotherapy starts, and the medicament passing member 43 is connected to the medicament storage member 41 and the medicament control member 42. After the to-be-irradiated body 200 determines a treatment plan, an operator in the medicament control room opens the medicament control member 42, and a doctor removes the syringe needle protection sleeve and inserts the syringe needle into the to-be-irradiated body 200. After a concentration of the boron (B-10)-containing medicine in the to-be-irradiated body 200 reaches a particular value, the to-be-irradiated body 200 may be transferred from the medicament control room 104 to the irradiation room 101. After the to-be-irradiated body 200 is transferred to a treatment location, the medicament passing member 43 is sequentially embedded into the first container 441 and the second container 442 of the containing mechanism 44. After the to-be-irradiated body 200 is positioned in the irradiation room 101 and the doctor leaves the irradiation room 101, the operator controls the neutron beam N to be irradiated on the to-be-irradiated body 200 and continues to control supplying of the boron(B-10)-containing medicine. It can be understood that the medicament supply apparatus 40 may alternatively be applied to a neutron capture therapy system of another type, and the boron(B-10)-containing medicine may alternatively be replaced with another medicine. In the process of transferring the to-be-irradiated body 200 from the medicament control room 104 to the irradiation room 101, the medicament passing member 43 does not need to be disconnected, and continuous medicament supplying may be implemented in the treatment process, thereby facilitating treatment and improving treatment efficiency.

The technical features in the foregoing embodiments may be randomly combined. For concise description, not all possible combinations of the technical features in the embodiments are described. However, provided that combinations of the technical features do not conflict with each other, the combinations of the technical features are considered as falling within the scope described in this specification.

The foregoing embodiments merely express several implementations of the present invention. The descriptions thereof are relatively specific and detailed, but are not understood as limitations on the scope of the present invention. It should be pointed out that a person of ordinary skill in the art can also make several transformations and improvements without departing from the idea of the present invention. These transformations and improvements fall within the protection scope of the present invention. Therefore, the protection scope of the patent of the present invention shall be subject to the appended claims.

## Claims

1. A medicament supply apparatus applied to a neutron capture therapy system, wherein the medicament supply apparatus comprises:
a medicament passing member, configured to supply a medicament to a to-be-irradiated body; and
a containing mechanism, configured to contain the medicament passing member, the containing mechanism comprising a first container arranged on a partition wall.

2. The medicament supply apparatus according to claim 1, wherein the partition wall comprises a first wall and a second wall formed with a slot body, and the first wall and the second wall are not in a same plane.

3. The medicament supply apparatus according to claim 2, wherein the first wall comprises a first wall portion; the second wall comprises a second wall portion formed with a first slot; the first wall portion is connected to the second wall portion; the first container comprises a first containing portion arranged at the first wall portion, and a second containing portion arranged in the first slot; and one end of the first containing portion is connected to one end of the second containing portion.

4. The medicament supply apparatus according to claim 3, wherein the second wall further comprises a third wall portion formed with a second slot, and a fourth wall portion formed with a third slot; the second wall portion, the third wall portion, and the fourth wall portion are connected to form a U-shaped structure; the first container further comprises a third containing portion arranged in the second slot, and a fourth containing portion arranged in the third slot; another end of the second containing portion is connected to one end of the third containing portion; and another end of the third containing portion is connected to one end of the fourth containing portion.

5. The medicament supply apparatus according to claim 4, wherein the first wall further comprises a fifth wall portion connected to the fourth wall portion, and the fourth wall portion and the fifth wall portion are not in a same plane; the first container further comprises a fifth containing portion arranged at the first wall portion, and a sixth containing portion arranged at the fifth wall portion; the fifth containing portion extends in a direction towards the second container; one end of the fifth containing portion is connected to another end of the first containing portion; and another end of the fourth containing portion is connected to one end of the sixth containing portion.

6. The medicament supply apparatus according to claim 4, wherein an upper side of the third containing portion further comprises a convex edge part arranged on an inner side of the fourth wall portion, and extends in a height direction of the fourth wall portion.

7. The medicament supply apparatus according to any one of claims 1 to 6, wherein the first container further comprises a stop part; the stop part allows the medicament passing member to be placed and prevents the medicament passing member from leaving the first container without an external force.

8. The medicament supply apparatus according to claim 7, wherein the stop part is correspondingly arranged on a corresponding containing portion, and at least a portion of the stop part extends in a direction from one side edge to another side edge of the containing portion.

9. The medicament supply apparatus according to claim 1, wherein the containing mechanism comprises a second container arranged on a floor.

10. The medicament supply apparatus according to claim 9, wherein the second container comprises a linear containing portion and a linear covering portion configured to cover the linear containing portion.

11. The medicament supply apparatus according to claim 10, wherein the second container further comprises a curved containing portion; the curved containing portion is connected to the linear containing portion; and the second container further comprises a curved covering portion configured to cover the curved containing portion.

12. The medicament supply apparatus according to claim 11, wherein both the linear covering portion and the curved covering portion have at least two through holes.

13. The medicament supply apparatus according to claim 11, wherein the linear covering portion and the curved covering portion are provided with bosses having widths less than widths of containing cavities of the linear containing portion and the curved containing portion.

14. The medicament supply apparatus according to claim 11, wherein the linear covering portion and the curved covering portion are made of neutron shielding materials.

15. The medicament supply apparatus according to claim 1, wherein the medicament supply apparatus further comprises a medicament control member and a medicament storage member; the medicament control member acts on the medicament passing member to control medicament supplying to the to-be-irradiated body; the medicament storage member stores a medicament required by the to-be-irradiated body and is connected to the medicament passing member; and the medicament control member and the medicament storage member are physically spatially separated from the second container.

16. A neutron capture therapy system, comprising:
a charged particle beam generation part, configured to generate a charged particle beam;
a neutron beam generation part, configured to generate a therapeutic neutron beam;
a beam transmission part, configured to transmit the charged particle beam to the neutron beam generation part;
an irradiation room, configured to perform neutron beam radiotherapy on an to-be-irradiated body;
a medicament control room, configured to control medicament supplying to the to-be-irradiated body; and
the medicament supply apparatus according to any one of claims 1 to 15, configured to supply a medicament to the to-be-irradiated body.
